# EUROPEAN PATENT SPECIFICATION

(11) **EP 3 292 882 B1**
(45) Date of publication and mention of the grant of the patent: **10.06.2020**
(21) Application number: 16188600.7
(22) Date of filing: 13.09.2016
(51) Int. Cl.: A61M 37/00, A61B 8/08, A61K 9/00, A61K 49/22, A61N 7/00

(54) **ULTRASONIC DEVICE FOR TRANSVERSELY MANIPULATING DRUG DELIVERY CARRIERS**
ULTRASCHALLVORRICHTUNG ZUR TRANSVERSALEN MANIPULATION VON ARZNEIMITTELABGABETRÄGERN
DISPOSITIF À ULTRASONS POUR MANIPULATION TRANSVERSALE DE SUPPORTS D'ADMINISTRATION DE MÉDICAMENT

(43) Date of publication of application: 14.03.2018
(73) Proprietor: National Tsing Hua University, Hsinchu (TW)
(72) Inventor: Lo, Wei-Chen, 970 Hualien County (TW); Yeh, Chih-Kuang, Hsinchu (TW); Kang, Shih-Tsung, 22048 New Taipei City (TW); Hsieh, Zong-Han, 413 Taichung City (TW)
(74) Representative: Lang, Christian

(56) References cited:
- WO-A1-2013/140175
- US-A- 6 029 518
- US-A1- 2013 046 229
- US-A1- 2016 250 457

## Description

### BACKGROUND

### Technical Field

The present disclosure relates to an ultrasonic device. More particularly, the present disclosure relates to an ultrasonic device for transversely manipulating drug delivery carriers.

### Description of Related Art

In present medical technology, delivering the drug to a lesion zone without passing the metabolism of the digestive system and the liver to maintain the concentration of the drug in the blood is a concerned research subject. However, it is difficult to deliver the drug to the lesion zone directly.

A drug delivery carrier is any substrate used in the process of drug delivery which serves to improve the selectivity, effectiveness, and/or safety of drug administration. Drug delivery carriers are primarily used to control the release of a drug into systemic circulation. This can be accomplished either by slow release of the drug over a long period of time or by triggered release at the drug's target by some stimulus, such as changes in pH, application of heat, and activation by light. However, the drug delivery carriers still cannot be delivered preciously so as to influence a local concentration of the drug delivery carriers at the lesion zone.

Manipulation of drug delivery carriers, such as microbubbles, cells and droplets, based on acoustic wave has become significant interest in biological and biomedical research due to their non-contact and non-invasive characters. The gas-filled microbubbles are encapsulated by an elastic shell and have great potential applications in drug delivery and targeted imaging. Transportation and trapping of the microbubbles to desired positions can improve the local concentration of the microbubbles in targeted areas and provide more efficient bonding.

One of current methods for manipulating the microbubbles is performed by a standing acoustic wave. The standing acoustic wave has become a powerful and active strategy to levitate or manipulate single or multiple particles and even living animals. However, forming the standing wave pattern has the nature of mandatory and environment dependent, and thus that limits the possibilities to manipulate an object in real applications. Another one of the current methods for manipulating the microbubbles is performed by a single-beam acoustic tweezer with a high frequency of more than 40MHz. However, the high frequency is not suitable to be applied on the human body.

US 2016/250457 A1 discloses an ultrasonic method and a device for cosmetic applications.

US 6,029,518 A describes an apparatus and a process for manipulating a body in a liquid mass with a plurality of acoustic radiation transducers. The wave front of the composite acoustical wave formed by the plurality of acoustic radiation transducers is an arc-shaped wave front. The freedom of manipulation of the body in the liquid mass is limited.

WO 2013/140175 A1 discloses an apparatus and a method for manipulating particles entrained in a fluid with an ultrasound transmitter comprising at least first and second groups of transmitter elements, whereat a region of minimum pressure is generated. The region of minimum pressure extends from the center point between the first and second groups of transmitter elements outwardly from the first and second groups of transmitter elements. The freedom of manipulation of the particles entrained in the fluid is also limited.

### SUMMARY

The present disclosure provides an ultrasonic device for transversely manipulating drug delivery carriers with the features of claim 1. Further embodiments are subject-matter of the dependent claims. The ultrasonic device includes a driving unit and a transducer. The transducer is electrically connected to the driving unit and has a piezoelectric sheet in a curved shape. The piezoelectric sheet includes a plurality of channels, and a phase difference is generated between every two of the channels by the driving unit for producing an acoustic vortex.

According to the ultrasonic device for transversely manipulating drug delivery carriers of the present disclosure, the piezoelectric sheet has a curvature radius ranged from 10 mm to 100 mm. In particular, the piezoelectric sheet is made of lead zirconate titanate.

According to the ultrasonic device for transversely manipulating drug delivery carriers of the present disclosure, the transducer further comprises a case. The case is provided for sealing the piezoelectric sheet and filled with epoxy.

According to the ultrasonic device for transversely manipulating drug delivery carriers of the present disclosure, the driving unit comprises a pulse generator.

According to the ultrasonic device for transversely manipulating drug delivery carriers of the present disclosure, the drug delivery carriers comprise a plurality of microbubbles.

According to the ultrasonic device for transversely manipulating drug delivery carriers of the present disclosure, the pulse generator is operated with a frequency ranging from 3 MHz to 20 MHz.

Moreover, the pulse generator is operated with a duty cycle of 30 % or above.

According to the ultrasonic device for transversely manipulating drug delivery carriers of the present disclosure, the phase difference ranges from π/8 to π/2.

According to the ultrasonic device for transversely manipulating drug delivery carriers of the present disclosure, an average particle size of the microbubbles ranges from 1 µm to 200 µm, and each of the microbubbles is a phospholipid-coated microbubble. Moreover, each of the microbubbles includes an ultrasound contrast agent or a drug.

### BRIEF DESCRIPTION OF THE DRAWINGS

The present disclosure can be more fully understood by reading the following detailed description of the embodiment, with reference made to the accompanying drawings as follows:
Fig. 1A is a schematic drawing of an ultrasonic device for transversely manipulating drug delivery carriers according to one embodiment of the present disclosure;
Fig. 1B is a schematic drawing of a piezoelectric sheet according to one embodiment of the present disclosure;
Fig. 2A is a flow chart of a method for transversely manipulating drug delivery carriers according to one embodiment of the present disclosure;
Fig. 2B is a schematic drawing showing an operation mode of the ultrasonic device in Fig. 1A;
Fig. 2C is a schematic drawing showing the method for transversely manipulating drug delivery carriers of Step S204 in Fig. 2A;
Fig. 3A is an axial measured result of a vortex acoustic field according to Example 1 of the present disclosure;
Fig. 3B is a transverse measured result of the vortex acoustic field of Fig, 3A;
Fig. 4A is an image showing microbubbles in a needle hydrophone without applying the acoustic vortex according to Example 1 of the present disclosure;
Figs. 4B-4D are images showing the microbubbles of Fig. 4A with applying the acoustic vortex;
Fig. 4E is an image showing a trajectory of a microbubbles cluster manipulated by the ultrasonic device of Fig. 1A;
Fig. 5A is an image showing a counterclockwise manipulation of a microbubbles cluster;
Fig. 5B is an image showing a clockwise manipulation of a microbubbles cluster; and
Fig. 6 is an image showing a manipulation of microbubbles in a needle hydrophone with a high flow rate according to Example 2 of the present disclosure.

### DETAILED DESCRIPTION

The present disclosure provides an ultrasonic device for transversely manipulating drug delivery carriers and allows collect and manipulation microbubbles in a desired position. The low frequency, appropriate working distance for veins, arteries or a deep tissue of the human body, and single-beam configuration provide superior usefulness compared with the conventional methods particularly in drug delivery applications.

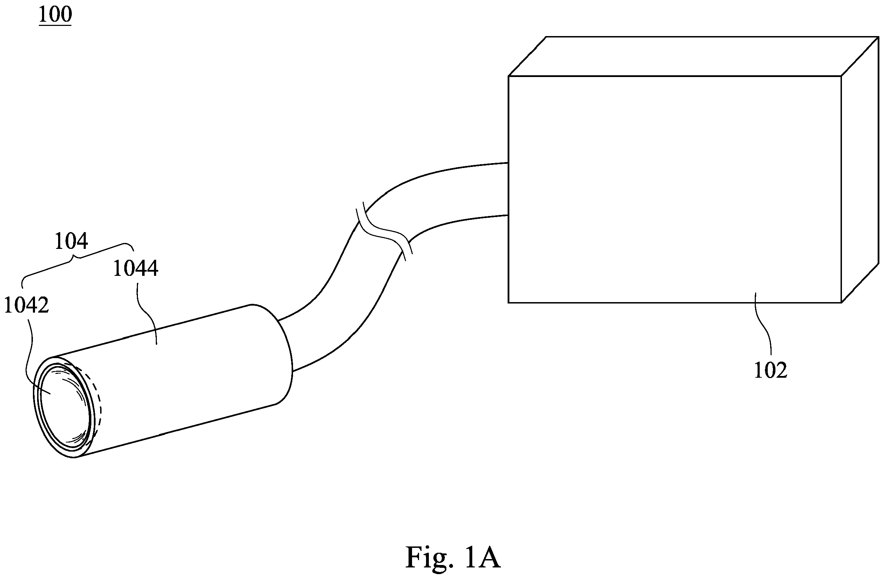

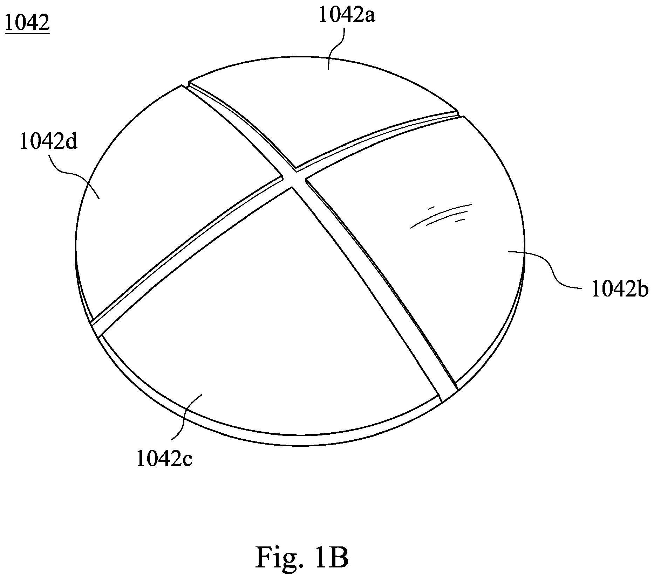

Please refer to Fig. 1A and Fig. 1B. Fig. 1A is a schematic drawing of an ultrasonic device 100 for transversely manipulating drug delivery carriers according to one embodiment of the present disclosure, and Fig. 1B is a schematic drawing of a piezoelectric sheet 1042 according to one embodiment of the present disclosure. As shown in Fig. 1A, the ultrasonic device 100 for transversely manipulating drug delivery carriers at least includes a driving unit 102 and a transducer 104. The transducer 104 is electrically connected to the driving unit 102. Furthermore, the transducer 104 has a piezoelectric sheet 1042, and the piezoelectric sheet 1042 is in a curved shape and includes a plurality of channels (please refer to Fig. 1B). A phase difference is generated between every two of the channels by the driving unit for producing an acoustic vortex (not shown in the figure).

In particular, the driving unit 102 can be a pulse generator. More particularly, the driving unit 102 can be but not limited to a field-programmable gate array (FPGA)-based pulse generator. In addition, a driving signal transmitted by the driving unit 102 can be a square-wave signal or a sine wave signal. Although an amplifier is not shown in the figure, the amplifier can be disposed between the driving unit 102 and the transducer 104 for amplifying the driving signal.

In particular, the transducer 104 can be an array-based transducer. Thus, as shown in Fig. 1B, the piezoelectric sheet 1042 is cut into four adjacent channels, that is, channel 1042a, channel 1042b, channel 1042c and channel 1042d. In addition, the channel 1042a, channel 1042b, channel 1042c and channel 1042d can be obtained by a laser cutting method so as to maintain characteristics of the piezoelectric sheet 1042. However, the present disclosure is not limited thereto. The piezoelectric sheet 1042 also can be cut into eight adjacent channels.

In details, the transducer 104 further includes a case 1044 for sealing the piezoelectric sheet 1042 therein. According to one embodiment of the present disclosure, the piezoelectric sheet 1042 is made of lead zirconate titanate (PZT), and the case 1044 is made of acrylic material. Moreover, the case 1044 can be filled with epoxy, but the present disclosure is not limited thereto.

It is noted that the piezoelectric sheet 1042 has a curvature radius ranged from 10 mm to 100 mm. In details, a focal length of the piezoelectric sheet 1042, that is, the working distance of the transducer 104, is ranged from 10 mm to 100 mm. More particularly, the curvature radius of the piezoelectric sheet 1042 is ranged from 10 mm to 30 mm. Such the working distance of the transducer 104 is short enough to be applied in the veins or arteries of the human body, a micro-electro-mechanical system, or a microscopic scale.

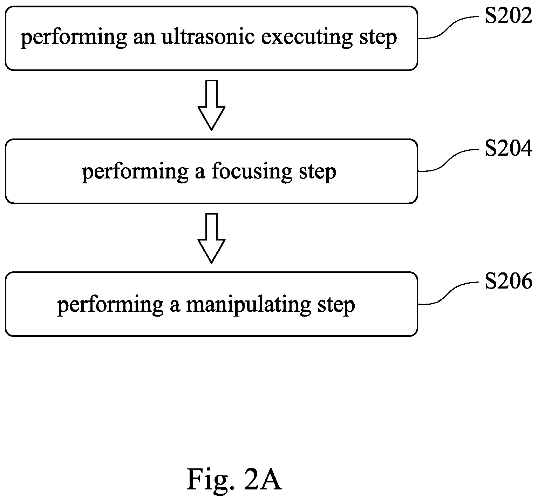

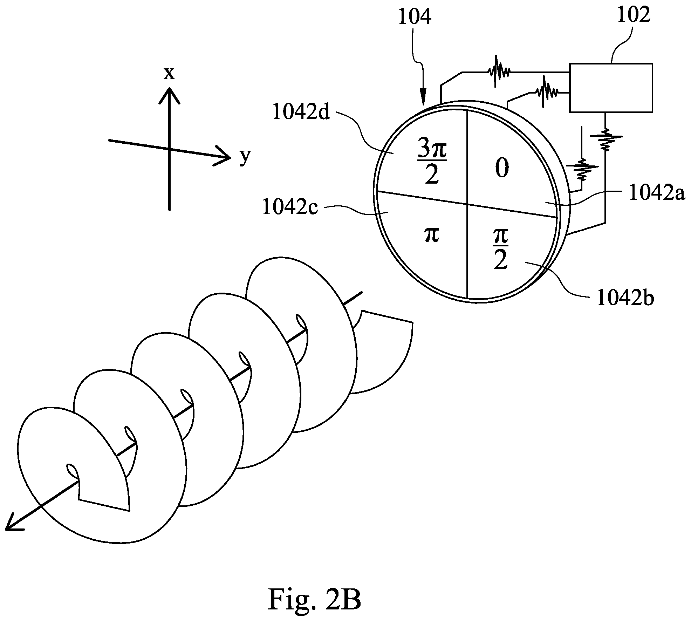

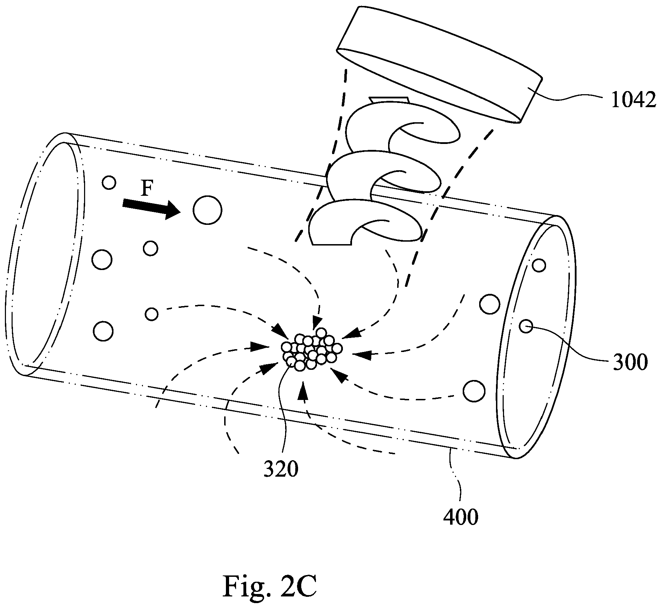

Please refer to Fig. 2A, Fig. 2B and Fig. 2C. Fig. 2A is a flow chart of a method for transversely manipulating drug delivery carriers not forming part of the invention, Fig. 2B is a schematic drawing showing an operation mode of the ultrasonic device 100 in Fig. 1A, and Fig. 2C is a schematic drawing showing the method for transversely manipulating drug delivery carriers of Step S204 in Fig. 2A. As shown in Fig. 2A, not forming part of the present invention is a method for transversely manipulating drug delivery carriers, and the method includes Step S202, Step S204 and Step S206.

Step S202 is an ultrasonic executing step. As mentioned above, the transducer 104 of the present disclosure can adopt the piezoelectric sheet 1042 with four channels as shown in Fig. 2B. It is noted that the piezoelectric sheet 1042 of the present disclosure needs to be in the curved shape although it seems a planar sheet in Fig. 2B for further illustration. In Step S202, the piezoelectric sheet 1042 can be driven with π/2-rad phase difference along the beam axis to produce an acoustic vortex. That is, the phase difference of π/2 is generated between every two of the channel 1042a, channel 1042b, channel 1042c and channel 1042d. However, the abovementioned phase difference can be ranged from π/8 to π/2 and is not limited thereto. Accordingly, each channel could be manually controlled so that the present disclosure has less limitation for application.

Step S204 is a focusing step. As shown in Fig. 2C, for simulating the application in the human body, the drug delivery carriers 300 can be suspended in a vessel phantom 400, such as a vein phantom or an artery phantom, and flow along with a direction F of a fluid filled in the vessel phantom 400. Thus, Step S204 is provided for creating a steep potential distribution in a center of the vortex so as to focus the drug delivery carriers 300 toward the center of the acoustic vortex to form a microbubbles cluster 320.

Step S206 is a manipulating step for manipulating the drug delivery carriers 300 to a lesion zone (not shown in the figure).

In details, Step S202 is performed by the driving unit, preferably a pulse generator, with a frequency ranged from 3 MHz to 20 MHz, preferably from 3 MHz to 5 MHz. Accordingly, such the low frequency is suitable to be applied in the human body. Furthermore, Step S202 is performed by the pulse generator with a duty cycle of 30 % or above.

Moreover, the drug delivery carriers of the present disclosure are a plurality of microbubbles. In particular, an average particle size of the microbubbles is ranged from 1 µm to 200 µm. Furthermore, each of the microbubbles comprises an ultrasound contrast agent or a drug. It is noted that each of the microbubbles is an elastomer and can generate a cavitation with the acoustic vortex so as to be controlled by a radiation force.

The ultrasonic device for transversely manipulating drug delivery carriers and the exemplary method using the same have been described as mentioned above. In the following, Example 1 and example 2 will be further provided to illustrate transmit conditions of the abovementioned ultrasonic device 100, the method using the same, and the effects of the present disclosure in details.

### <Example 1>

In Example 1, the driving unit is a FPGA-based pulse generator a phase shift of 2π. The vortex acoustic field generated is measured at two different observation planes using a 200 µm needle hydrophone (HG-0085, Onda, Sunnyvale, USA) mounted on a 3-D computer controlled motor system. Herein, the needle hydrophone is used as a vessel phantom, such as a vein phantom or an artery phantom, for simulating the application in the human body. Furthermore, the piezoelectric sheet of the transducer has four channels. More particularly, a .curvature radius of the piezoelectric sheet is 20 mm.

In Example 1, each of the microbubbles is used as the drug delivery carrier, respectively, and can be a phospholipid-coated microbubble. More particularly, the microbubbles are fabricated by using the compositions of 1,2-dipalmitoyl-sn-glycero-3-phosphocholine (DPPC), 1,2-distearoyl-sn-glycero-3-phospho-(1'-rac-glycerol) (DSPG), and 1,2-distearoyl-sn-glycero-3-phosphoethanolamine-N-[methoxy(polyethylene glycol)-5000] (DEPE-PEG5000). However, the present disclosure is not limited thereto.

In brief, the center of the acoustic vortex where all phases are perfect destructive interference results from the beam axis so as to form a potential well. In Example 1, the microbubbles are exposed to the acoustic vortex at one fourth the Rayleigh distance (RD/4) from the transducer. When the microbubbles are subjected into a fluid of the needle hydrophone and the vortex acoustic field is applied, the oscillating pressure gradient can couple with the bubble oscillations to produce the radiation force. Thus, each of the microbubbles will be trapped at the potential well and then transported. The motion of each microbubble can be recorded with B-mode imaging using a clinical ultrasound imaging system (model t3000, Terason, USA), however, the present disclosure is not limited thereto. Other transmit parameters of the driving unit are listed in Table 1.

**Table 1**

| | |
|---|---|
| Frequency (MHz) | 3 |
| Waveform | Sinusoid |
| Pulse duration (cycle) | 1000 |
| Duty cycle (%) | 33 |
| Acoustic pressure (kPa) | 40 |

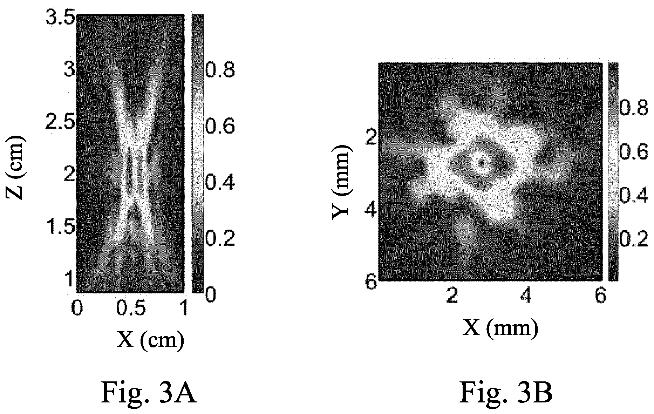

Please refer to Fig. 3A and Fig. 3B. Fig. 3A is an axial measured result of a vortex acoustic field according to Example 1 of the present disclosure, and Fig. 3B is a transverse measured result of the vortex acoustic field of Fig. 3A. As shown in Fig. 3A, the focal length of the vortex acoustic filed is 2 cm. That is, the manipulation of the microbubbles using the ultrasonic device provided in Example 1 has a shorter working distance so as to be applied in the micro-electro-mechanical system or the microscopic scale easily. Furthermore, the acoustic vortex refers to a type of beam having a helicoidal wave front and hence, the beam has a central dark core of zero amplitude surrounded by a high intensity ring as shown in Fig. 3B.

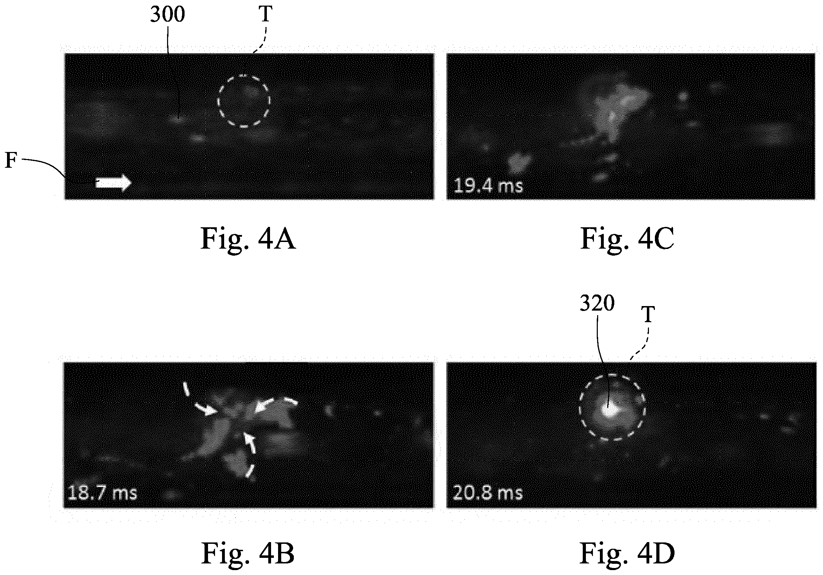

Please refer to Figs. 4A∼4D. Fig. 4A is an image showing microbubbles 300 in a needle hydrophone without applying the acoustic vortex according to Example 1 of the present disclosure, and Figs. 4B∼4D are images showing the microbubbles 300 of Fig. 4A with applying the acoustic vortex. As shown in Fig. 4A, the microbubbles 300 are suspended in the fluid of the needle hydrophone and flow along the direction F of the fluid, which has a volume velocity of 0.5 mL/hr, when the ultrasonic device is not applied and the acoustic vortex has not been produced yet. When the ultrasonic device is applied on a target zone T, which is represented by a dotted line, to produce the acoustic vortex, the microbubbles 300 start to swirling toward a center of the acoustic vortex as shown in Fig. 4B and Fig. 4C. Finally, a microbubbles cluster 320 will be formed in the target zone T as shown in Fig. 4D.

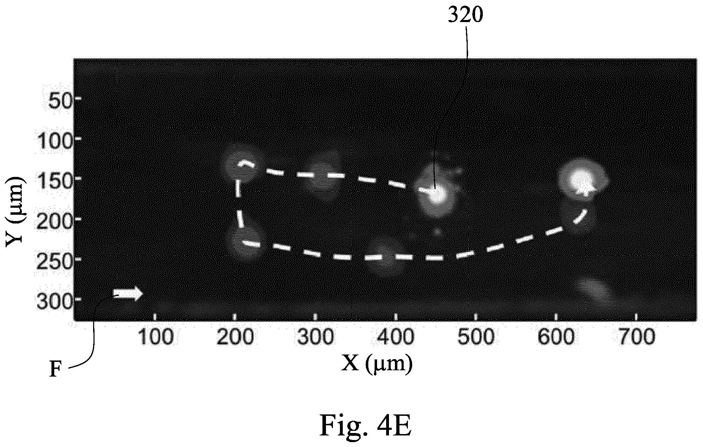

Please refer to Fig. 4E, which is an image showing a trajectory of the microbubbles cluster 320 manipulated by the ultrasonic device 100 of Fig. 1A. As shown in Fig. 4E, the microbubbles cluster 320 can be manipulated to move along with the motions of the transducer. That is, the acoustic vortex provides the capability of 2-D particles manipulation.

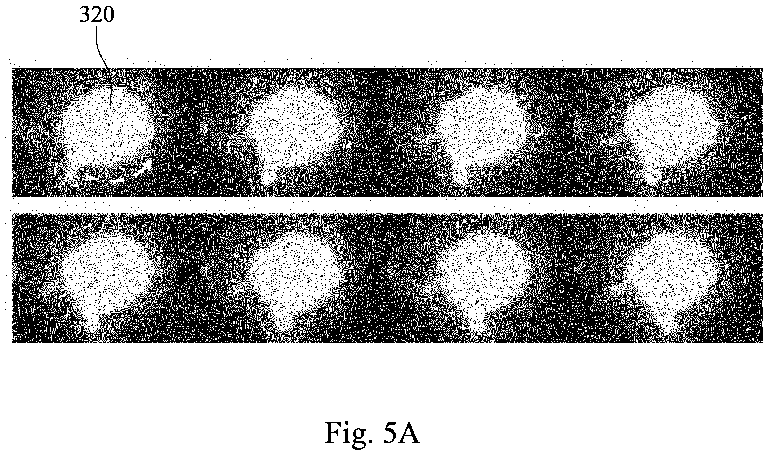

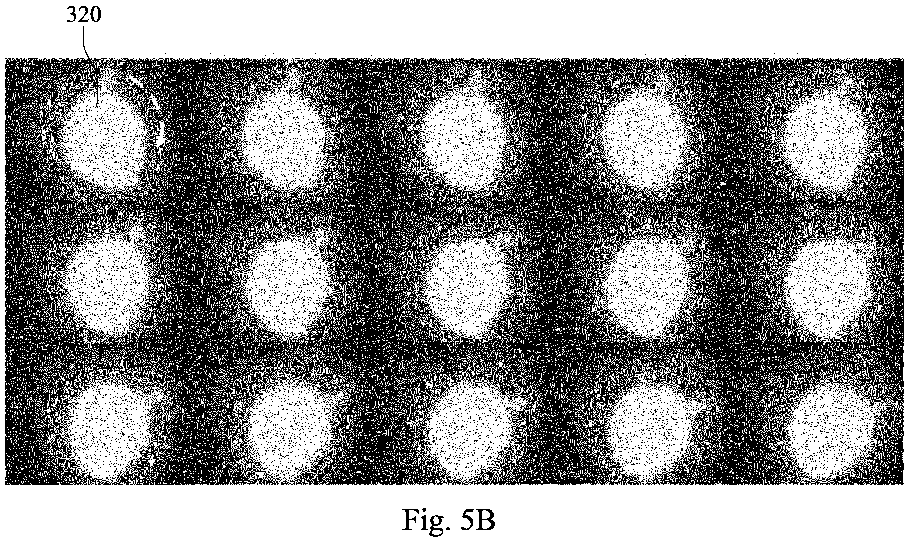

Please further refer to Fig. 5A and Fig. 5B. Fig. 5A is an image showing a counterclockwise manipulation of the microbubbles cluster 320, and Fig. 5B is an image showing a clockwise manipulation of the microbubbles cluster 320. In Fig. 5A, from left to right and from up to down, the microbubbles cluster 320 can be manipulated to perform a counterclockwise rotation. In Fig. 5B, from left to right and from up to down, the microbubbles cluster 320 also can be manipulated to perform a clockwise rotation. That is to say, except for the transportation, the acoustic vortex of the present disclosure also can manipulate the microbubbles cluster to rotate.

### <Example 2>

Example 2 is provided for simulating the manipulation of the microbubbles under a high flow rate, for example, the manipulation in the artery. The ultrasonic device and the method for manipulating the drug delivery carriers of Example 2 are similar to Example 1 except the transmit parameters. The transmit parameters are further listed in Table 2.

**Table 2**

| | |
|---|---|
| Frequency (MHz) | 3 |
| Waveform | Sinusoid |
| Pulse duration (cycle) | 1000 |
| Duty cycle (%) | 99 |
| Acoustic pressure (kPa) | 800 |

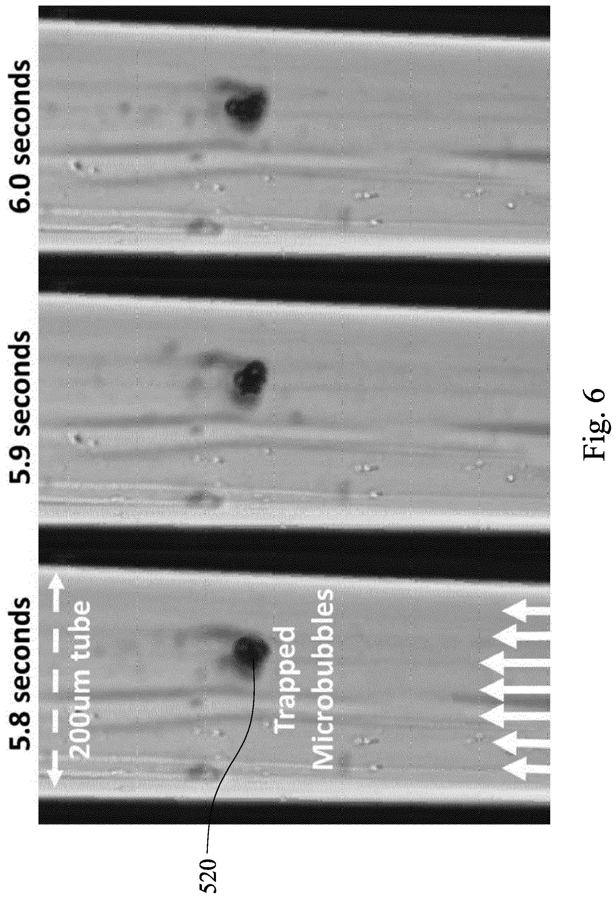

Please refer to Fig. 6, which is an image showing a manipulation of microbubbles in a needle hydrophone with a high flow rate according to Example 2 of the present disclosure. In Example 2, it is noted that the microbubbles (not shown in the figure) still can be trapped to form a microbubbles cluster 520 even though the flow rate of the fluid in the needle hydrophone is 5 mL/hr. That is, the ultrasonic device and the method for manipulating the drug delivery carriers disclosed in the present disclosure can be applied but not limited to the artery, where has a high flow rate of blood, of the human body.

To sum up, the present disclosure provides an ultrasonic device for transversely manipulating drug delivery carriers and allows collect and manipulation microbubbles in a desired position. The low frequency, appropriate working distance for veins, arteries or a deep tissue of the human body, and single-beam configuration provide superior usefulness compared with the conventional methods particularly in drug delivery applications. Moreover, the trapping characteristics may be useful to increase the efficiency of microbubbles accumulation at the lesion zone.

## Claims

1. An ultrasonic device (100) for transversely manipulating drug delivery carriers (300), comprising:
a driving unit (102); and
a transducer (104) electrically connected to the driving unit (102) and having a piezoelectric sheet (1042) in a curved shape, wherein the piezoelectric sheet (1042) comprises a plurality of channels (1042a, 1042b, 1042c, 1042d),
**characterised in that**
a phase difference is generated between every two of the channels (1042a, 1042b, 1042c, 1042d) by the driving unit (102) for producing an acoustic vortex.

2. The ultrasonic device (100) of claim 1, wherein the piezoelectric sheet (1042) has a curvature radius ranged from 10 mm to 100 mm.

3. The ultrasonic device (100) of claim 1, wherein the piezoelectric sheet (1042) is made of lead zirconate titanate.

4. The ultrasonic device (100) of claim 1, wherein the transducer (104) further comprises:
a case (1044) provided for sealing the piezoelectric sheet (1042) and filled with epoxy.

5. The ultrasonic device (100) of claim 1, wherein the driving unit (102) comprises a pulse generator.

6. The ultrasonic device (100) of claim 1, wherein the drug delivery carriers (300) comprise a plurality of microbubbles (300).

7. The ultrasonic device (100) of claim 5, wherein the pulse generator is operated with a frequency ranging from 3 MHz to 20 MHz.

8. The ultrasonic device (100) of claim 5, wherein the pulse generator is operated with a duty cycle of 30 % or above.

9. The ultrasonic device (100) of claim 1, wherein the phase difference ranges from π/8 to π/2.

10. The ultrasonic device (100) of claim 6, wherein an average particle size of the microbubbles (300) ranges from 1 µm to 200 µm.

11. The ultrasonic device (100) of claim 6, wherein each of the microbubbles (300) is a phospholipid-coated microbubble.

12. The ultrasonic device (100) of claim 6, wherein each of the microbubbles (300) comprises an ultrasound contrast agent or a drug.

## Patentansprüche

1. Ultraschallvorrichtung (100) zur transversalen Manipulation von Arzneimittelabgabeträgem (300), umfassend:
eine Antriebseinheit (102) und
einen Wandler (104), der elektrisch mit der Antriebseinheit (102) verbunden ist und eine piezoelektrische Platte (1042) in einer gebogenen Form aufweist, wobei die piezoelektrische Platte (1042) eine Mehrzahl an Kanälen (1042a, 1042b, 1042c, 1042d) aufweist,
**dadurch gekennzeichnet**, durch
eine Phasendifferenz zwischen jeweils zwei der Kanäle (1042a, 1042b, 1042c, 1042d) durch die Antriebseinheit (102) erzeugt wird, um einen akustischen Wirbel zu erzeugen.

2. Ultraschallvorrichtung (100) gemäß Anspruch 1, bei der die piezoelektrische Platte (1042) einen Krümmungsradius im Bereich von 10 mm bis 100 mm aufweist.

3. Ultraschallvorrichtung (100) gemäß Anspruch 1, bei der die piezoelektrische Platte (1042) aus Blei-Zirkonat-Titanat hergestellt ist.

4. Ultraschallvorrichtung (100) gemäß Anspruch 1, bei der der Wandler (104) ferner umfasst:
ein Gehäuse (1044), das zum Verschließen der piezoelektrischen Platte (1042) bereitgestellt ist und mit Epoxid gefüllt ist.

5. Ultraschallvorrichtung (100) gemäß Anspruch 1, bei der die Antriebseinheit (102) einen Pulsgenerator umfasst.

6. Ultraschallvorrichtung (100) gemäß Anspruch 1, bei der die Arzneimittelabgabeträger (300) eine Mehrzahl an Mikrobläschen (300) umfassen.

7. Ultraschallvorrichtung (100) gemäß Anspruch 5, bei der der Pulsgenerator mit einer Frequenz betrieben wird, die im Bereich von 3 MHz bis 20 MHz liegt.

8. Ultraschallvorrichtung (100) gemäß Anspruch 5, bei der der Pulsgenerator mit einem Auslastungsgrad von 30% oder höher betrieben wird.

9. Ultraschallvorrichtung (100) gemäß Anspruch 1, bei der die Phasendifferenz im Bereich von *π*/8 bis *π*/2 liegt.

10. Ultraschallvorrichtung (100) gemäß Anspruch 6, bei der eine durchschnittliche Partikelgröße der Mikrobläschen (300) im Bereich von 1 µm bis 200 µm liegt.

11. Ultraschallvorrichtung (100) gemäß Anspruch 6, bei der jedes der Mikrobläschen (300) ein Phospholipid-beschichtetes Mikrobläschen ist.

12. Ultraschallvorrichtung (100) gemäß Anspruch 6, bei der jedes der Mikrobläschen (300) ein Ultraschallkontrastmittel oder einen Wirkstoff umfasst.

## Revendications

1. Dispositif à ultrasons (100) pour manipulation transversale de supports d'administration de médicament (300), comprenant :
une unité d'entraînement (102) ; et
un transducteur (104) relié électroniquement à l'unité d'entraînement (102) et présentant une feuille piézoélectrique (1042) dans une forme incurvée, dans lequel la feuille piézoélectrique (1042) comprend une pluralité de canaux (1042a, 1042b, 1042c, 1042d),
**caractérisé en ce qu'**
une différence de phase est générée entre chaque paire de canaux (1042a, 1042b, 1042c, 1042d) par l'unité d'entraînement (102) pour produire un vortex acoustique.

2. Dispositif à ultrasons (100) selon la revendication 1, dans lequel la feuille piézoélectrique (1042) présente un rayon de courbure dans la plage allant de 10 mm à 100 mm.

3. Dispositif à ultrasons (100) selon la revendication 1, dans lequel la feuille piézoélectrique (1042) est faite de titano-zirconate de plomb.

4. Dispositif à ultrasons (100) selon la revendication 1, dans lequel le transducteur (104) comprenant en outre :
un boîtier (1044) prévu pour sceller la feuille piézoélectrique (1042) et rempli d'époxy.

5. Dispositif à ultrasons (100) selon la revendication 1, dans lequel l'unité d'entraînement (102) comprend un générateur d'impulsions.

6. Dispositif à ultrasons (100) selon la revendication 1, dans lequel les supports d'administration de médicament (300) comprennent une pluralité de microbulles.

7. Dispositif à ultrasons (100) selon la revendication 5, dans lequel le générateur d'impulsions fonctionne avec une fréquence dans une plage allant de 3 MHz à 20 MHz.

8. Dispositif à ultrasons (100) selon la revendication 5, dans lequel le générateur d'impulsions fonctionne selon un cycle de travail de 30 % ou plus.

9. Dispositif à ultrasons (100) selon la revendication 1, dans lequel la différence de phase est dans une plage allant de Π/8 à Π/2.

10. Dispositif à ultrasons (100) selon la revendication 6, dans lequel une taille de particule moyenne des microbulles (300) est dans la plage de 1 µm à 200 µm.

11. Dispositif à ultrasons (100) selon la revendication 6, dans lequel chacune des microbulles (300) est une microbulle revêtue d'un phospholipide.

12. Dispositif à ultrasons (100) selon la revendication 6, dans lequel chacune des microbulles (300) comprend un agent de contraste ultrasonore ou un médicament.
